(19) Europäisches Patentamt
European Patent Office
Office européen des brevets

(11) EP 3 930 670 B1

(12) # EUROPÄISCHE PATENTSCHRIFT

(45) Veröffentlichungstag und Bekanntmachung des Hinweises auf die Patenterteilung:
**05.04.2023 Patentblatt 2023/14**

(21) Anmeldenummer: **20704897.6**

(22) Anmeldetag: **18.02.2020**

(51) Internationale Patentklassifikation (IPC):
***A61K 8/37*** (2006.01) ***A61Q 5/00*** (2006.01)
***A61Q 5/02*** (2006.01) ***A61Q 19/00*** (2006.01)
***A61Q 19/10*** (2006.01) ***A61K 8/92*** (2006.01)
***A61K 8/04*** (2006.01)

(52) Gemeinsame Patentklassifikation (CPC):
**A61K 8/375; A61K 8/04; A61K 8/922; A61Q 5/00; A61Q 5/02; A61Q 19/00; A61Q 19/10**

(86) Internationale Anmeldenummer:
**PCT/EP2020/054236**

(87) Internationale Veröffentlichungsnummer:
**WO 2020/173761 (03.09.2020 Gazette 2020/36)**

(54) **BIOBASIERTE PERLGLANZWACHSE**

BIO-BASED PEARLESCENT WAXES

CIRES NACRÉES BIOSOURCÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.02.2019 EP 19159688**

(43) Veröffentlichungstag der Anmeldung:
**05.01.2022 Patentblatt 2022/01**

(73) Patentinhaber: **BASF SE**
**67056 Ludwigshafen am Rhein (DE)**

(72) Erfinder:
- **STOER, Claudia**
  **40789 Monheim (DE)**
- **NIEENDICK, Claus**
  **40589 Düsseldorf-Holthausen (DE)**
- **DIERKER, Markus**
  **40589 Düsseldorf-Holthausen (DE)**
- **BEHLER, Ansgar**
  **40589 Düsseldorf-Holthausen (DE)**
- **CORNELSEN, Sybille**
  **40789 Monheim (DE)**
- **MAUER, Werner**
  **40589 Düsseldorf-Holthausen (DE)**

(74) Vertreter: **BASF IP Association**
**BASF SE**
**GBI-C006**
**67056 Ludwigshafen (DE)**

(56) Entgegenhaltungen:
**EP-A2- 0 376 083** **WO-A1-2018/089600**
**DE-A1- 19 511 572** **US-A1- 2015 335 550**

EP 3 930 670 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

### Gebiet der Erfindung

Die Erfindung liegt auf dem Gebiet spezieller wachsartiger Ester, die insbesondere in Form als Wachsdispersionen, eine Trübung und/oder einen Perlglanz in kosmetischen Reinigungsmitteln bewirken. Weitere Gegenstände betreffen die Verwendung der Wachse in kosmetischen Mitteln, deren Herstellung und die kosmetischen Mittel selber.

### Stand der Technik

Kosmetische Reinigungsmittel für Haut- und/oder Haar wie Haarshampoos, Haarkonditionierer, Duschgele oder flüssige Handseifen werden gerne optisch ansprechend formuliert. Man kann die kosmetischen Reinigungsmittel mit einem glänzenden Glimmereffekt, der meist auch als Perlglanz bezeichnet wird, oder mit einer nicht-glänzenden Milchfärbung, die als Trübung oder Weißfärbung bezeichnet wird, formulieren.

Häufig werden als Trübungsmittel für kosmetische Reinigungsmittel feinteilige Polymerdispersionen, meist auf Basis von Copolymerisaten von (Meth) Acryl und Styrol, verwendet. Aus ökologischen Gründen möchte man diese ersetzen.

Aus dem Deutschen Patent DE19511572 sind Trübungsmittelkonzentrate auf Basis von Wachskörpern mit einem hydrophilen und hydrophoben Emulgator bekannt. Demnach ergeben Ethylenglykoldistearat (Wachskörper) in Kombination mit einem Zuckertensid (hydrophiler Emulgator) und einem Monoglycerid (hydrophober Emulgator) niedrigviskose, trübe Wachsdispersionen. Das verwendete Ethylenglykoldistearat ist ein Fettsäureester, der auf petrochemisch hergestelltem Ethylenglykol basiert.

Aus dem Stand der Technik sind derartig petrochemisch basierte Wachse wie Ethylenglykolmonostearat (EGMS) und/oder -distearat (EGDS) auch als Wachskörper in Perlglanzmitteln bzw. Perlglanzkonzentraten bekannt. So wird gemäß der Internationalen Patentanmeldung WO98/38973 durch einen besonders hohen C18-Fettsäuregehalt oder gemäß der Internationalen Patentanmeldung WO2012/177886 durch die besondere Art der Kristallbildung ein sehr guter Perlglanzeffekt erreicht. Des Weiteren gibt es einen breiten Stand der Technik, mit welchen Mitteln man die Wachse EDMS und/oder EDGS in Wachsdispersionen überführen kann, beispielsweise gemäß der Internationalen Patentanmeldung WO03/066796 mittels nichtionischer Tenside.

Diese Wachse sind bislang ausschließlich Ester des Ethylenglykols auf petrochemischer Basis, d.h. fossilen Ursprungs.

Aufgabe der vorliegenden Erfindung war es unter anderem, Wachse für Wachsdispersionen zur Verfügung zu stellen, die dem Menschen ein nachhaltiges Verhalten mit seiner Umwelt ermöglicht. So sollten mindestens die Wachse vollständig pflanzenbasiert sein und möglichst die gesamte Wachsdispersion nicht auf petrochemischen bzw. fossilen Rohstoffen basieren.

Gleichzeitig sollten die Wachse in Form ihrer Wachsdispersionen aber weiterhin mindestens die gleichen Qualitäten als Perlglanz- oder Trübungsmittel, möglichst sogar noch bessere Qualitäten, aufweisen.

Die Aufgabe wurde gelöst durch wachsartige Ester des Ethylenglykols, insbesondere geeignet als Trübungs- und/oder Perlglanzmittel in kosmetischen Reinigungsmitteln, dadurch gekennzeichnet, dass das Ethylenglykol im Ester nach der $^{14}C$-Analytik mindestens 99% biogenen Anteil an Kohlenstoff aufweist, der gemäß DIN EN 15440 mit 13,6 dpm/gC matrixunabhängig vorgegeben ist; mit einer Standardabweichung von +/- 0 bis +/- 4.

Vorzugsweise weist das Ethylenglykol im Ester nach der $^{14}C$-Analytik 100% biogenen Anteil an Kohlenstoff auf, der gemäß DIN EN 15440 mit 13,6 dpm/gC matrixunabhängig vorgegeben ist; mit einer Standardabweichung von +/- 0 bis +/- 4.

Vollkommen überraschend zeigen diese wachsartigen Ester des Ethylenglykols mit dem biogenen Gehalt an $^{14}C$-Isotop in Form einer Wachsdispersion, einen besseren Weißheitsgrad als die bisherigen Ester auf Basis des petrochemischen bzw. fossilen Ethylenglykols. Dies war nicht zu erwarten, da der Anteil an $^{14}C$-Isotop im Ester des Ethylenglykols extrem niedrig ist.

Zudem zeigen die erfindungsgemäßen Ethylenglykolester eine niedrigere Schmelzenthalpie als vergleichbare petrochemische Ethylenglykolester, so dass für das Aufschmelzen einer definierten Menge an erfindungsgemäßen Wachse deutlich weniger Energie benötigt wird im Vergleich zur gleichen Masse der petrochemischen Ethylenglykolester. Dies ermöglicht eine wirtschaftlichere und Energie schonendere Herstellung der Wachsdispersionen und deren Verarbeitbarkeit im kosmetischen Mittel.

Isotope sind Nuklide gleicher Ordnungszahl (= Kernladungszahl, Atomnummer, Protonenzahl), aber unterschiedlicher Anzahl der im Kern enthaltenen Neutronen und damit unterschiedlicher Massenzahl [= Nukleonenzahl, Zahl der in einem Atomkern enthaltenen Nukleonen (Protonen und Neutronen)]. Isotope unterscheiden sich außer durch die Masse auch durch Drehimpuls (Kernspin), magnetisches Moment und elektrisches Quadrupolmoment.

Zur eindeutigen Kennzeichnung der Isotope benutzt man die für Nuklide allgemein gebräuchliche Schreibweise

$^{A}{}_{Z}X$ (X = chemisches Symbol, A = Massenzahl, Z = Kernladungszahl), für die stabilen Isotope des Kohlenstoffs also $^{12}{}_{6}C$, alternativ wird als Schreibweise $^{12}C$ oder C-12 verwendet.

Das Element Kohlenstoff hat insgesamt 2 stabile Isotope $^{12}C$ und $^{13}C$. $^{12}C$ kommt zu 98,9 % in der Natur vor, $^{13}C$ zu 1,1 %. Neben diesen beiden stabilen Isotopen gibt es noch mehrere instabile Isotope. Das bekannteste instabile Isotop ist dabei $^{14}C$ mit einer Halbwertszeit von 5730 Jahren. $^{14}C$ entsteht durch natürliche Kernreaktion in der Atmosphäre aus $^{14}N$: die Erde ist ständig kosmischer Strahlung ausgesetzt, trifft diese Strahlung auf die obersten Schichten der Erdatmosphäre erzeugt dies freie Neutronen. Diese wiederum reagieren mit dem in der unteren Atmosphäre zu etwa 80 % in der Luft enthaltenen Stickstoff. Dabei läuft folgende Reaktion ab:

$$^{14}N + {}^{1}n \rightarrow {}^{14}C + {}^{1}p$$

Der Kern eines Stickstoffatoms mit der Massezahl 14 (7 Neutronen, 7 Protonen) nimmt ein Neutron auf. Unter Abgabe eines Protons entsteht aus dem Stickstoffatom das radioaktive Kohlenstoffisotop $^{14}C$ (8 Neutronen, 6 Protonen), die Massezahl bleibt also gleich. Kohlenstoff $^{12}C$ hat dagegen 6 Neutronen und 6 Protonen - ist also leichter als $^{14}C$.

Der in der Atmosphäre erzeugte $^{14}C$ verbindet sich mit vorhandenem Sauerstoff zu Kohlendioxid. Durch die Photosynthese der Pflanzen gelangt $^{14}C$ so anschließend in die Biosphäre. Da Lebewesen bei ihrem Stoffwechsel ständig Kohlenstoff mit der Atmosphäre austauschen, stellt sich in lebenden Organismen dasselbe Verteilungsverhältnis der 3 Kohlenstoff-Isotope $^{12}C$, $^{13}C$ und $^{14}C$ ein, wie es in der Atmosphäre vorliegt: Lebende Organismen enthalten pro $10^{12}$ stabilen $^{12}C$- u. $^{13}C$-Isotopen ca. 1,2 radioaktive $^{14}C$-Isotope.

Wird Kohlenstoff aus diesem Kreislauf herausgenommen (d.h. wird er fossil), dann ändert sich das Verhältnis zwischen $^{14}C$ und $^{12}C$, da die zerfallenden $^{14}C$-Isotope nicht durch neue ersetzt werden.

Fossile Brennstoffe, wie Erdöl, Erdgas oder Kohle, sind vor über 100 Millionen Jahren entstanden, d.h. diese Brennstoffe enthalten keine $^{14}C$-Isotope mehr, da die ursprünglich vorhandenen $^{14}C$-Isotope zerfallen sind und keine neuen $^{14}C$-Isotope aufgenommen wurden. Demnach enthalten Kohlenwasserstoffe, die aus fossilen Quellen stammen, keine $^{14}C$-Isotope.

In einer bevorzugten Ausführungsform der Erfindung liegt der Anteil an $^{14}C$-Isotope im Verhältnis zu den stabilen $^{12}C$- und $^{13}C$-Isotopen im Bereich von $6 \times 10^{-13}$ bis $1{,}2 \times 10^{-12}$. Bezugsgröße sind alle im Ethylenglykol vorhandenen Kohlenwasserstoffe.

Der $^{14}C$-Gehalt einer Probe kann entweder durch Zählung der zerfallenden $^{14}C$-Isotope im Zählrohr (Zählrohr Methode nach Libby), im Flüssigkeits-Szintillations-Spektrometer oder durch Zählung der noch vorhandenen $^{14}C$-Isotope mit der Beschleuniger-Massenspektrometrie bestimmt werden. Mit der Beschleuniger-Massenspektrometrie (Abkürzung: AMS von Accelerator Mass Spectrometry) können $^{14}C$-Isotope mit Hilfe kernphysikalischer Meßmethoden im ppt- bis ppq-Bereich (von $10^{-12}$ bis $10^{-16}$) in kleinsten Probenmengen (Milligrammbereich) nachgewiesen werden. Die derzeitige 100%ige biogene Aktivität ist für 2018 gemäß DIN 15440 mit 13,6 dpm/gC matrixunabhängig vorgegeben.

Die erfindungsgemäßen Ester des Ethylenglykols sind herstellbar durch Veresterung des speziellen Ethylenglykols, welches die $^{14}C$-Isotope enthält. Derartiges Ethylenglykol, welches auch unter dem Namen "Bioethylenglykol" oder "Bio-Monoethylenglykol" oder auch kurz "Bio-MEG" bekannt ist, ist nach verschiedenen Verfahren zugänglich. Der Zusatz "Bio" bezieht sich auf die Herkunft der Rohstoffe aus nachwachsenden bzw. den pflanzlich basierten Rohstoffen. Nach einem der älteren Verfahren der India Glycols Ind., Indien, wird zunächst aus Zuckerrohr oder auch aus Zuckerrohrsirup der sogenannte "Bio-Ethanol" hergestellt. Bio-Ethanol selber ist chemisch gesehen Ethylalkohol. Nach dem Verfahren der India Glycols Ind. wird aus Bio-Ethanol das gewünschte Ethylenglykol ("Bio-MEG") hergestellt. Hierfür wird mittels katalysierter Dehydratation (katalysierter Wasserabspaltung) zunächst Ethylen, anschließend durch Oxidation Ethylenoxid hergestellt, welches schließlich durch Wasseraddition in das Bio-MEG überführt wird.

Nach einem weiteren Verfahren kann aus Zuckerrohrsaft bzw. Melasse oder Bagasse in Anwesenheit von Hefen, insbesondere der Pilze aus der Gruppe der Schlauchpilze (Ascomycetes), auf anaerobem Weg Bio-Ethanol hergestellt werden, welches in Ethen überführt wird und mittels Oxidation in Essig und anschließender Hydrierung zu Bio-MEG führt. Derart hergestelltes Bio-MEG wird auch häufig zur Herstellung von sog. PlantBottle™, also PET-Flaschen mit 30% Bio-MEG verwendet.

Aufgrund des ansteigenden Verbrauches werden in Kürze zwei weitere Anlagen zur Herstellung von Bio-MEG angefahren. Nach dem angekündigten Verfahren der Braskem S.A. und Haldor Topsoe A/S wird das MOSAIK™ Verfahren (MOnoSAcchairde Indurstrial Cracker) mit dem Haldor Topsoe™ Prozess kombiniert, so dass zunächst Zucker in kleinere Moleküle oxidiert und diese in Anwesenheit eines heterogenen Nichtedelmetall-Katalysators zu Ethylenglykol und Propylenglykol überführt werden.

Avantium Chemicals B.V. plant nach dem Mekong™ Verfahren Bio-MEG herzustellen, wobei dies in einem einstufigen Verfahren aus Mono- und Disacchariden mit Wasserstoff in Gegenwart von Katalysatoren und unter Wasserabspaltung erfolgen soll.

Prinzipiell kann Bio-MEG natürlich auch aus anderen nachwachsenden Rohstoffen wie Zuckerrohr oder Zuckerrüben hergestellt werden, beispielsweise aus stärkehaltigen Pflanzen wie Getreide, Kartoffeln und Mais oder aus zellulosehaltigen Rohstoffen wie Stroh und Holz basiert.

Bevorzugt werden Ester von Ethylenglykol auf Basis von Zuckerrohr oder Zuckerrüben, insbesondere in Form des Zuckersirups.

Derartige pflanzenbasierte Ethylenglykole sind beispielsweise erhältlich bei der Firma India Glycols Ltd, siehe auch http://www.indiaglycols.com/product_groups/monoethylene_glycol.htm.

Unter dem Begriff "Ethylenglykol" bzw. Monoethylenglykol wird idealerweise ein 1,2-Monoethylendiol verstanden, welches der allgemeinen Formel (**I**)

$$HOCH_2CH_2OH \qquad \textbf{(I)}$$

folgt.

In der Praxis enthalten chemisch hergestellte Verbindungen jedoch in verschiedenen Konzentrationen Beiprodukte. Im Sinne der Erfindung sind Ester basierend auf Ethylenglykol bevorzugt, die einen Reinheitsgrad von 99,7 bis 99,9 % aufweisen, d.h. 99,7 bis 99,9 Gew.-% sind Monoethylenglykol. Besonders bevorzugt sind solche Ethylenglykole, die in Mengen von max. 0,06 Gew.-% Wasser und restliche Katalysatormengen im ppm Bereich haben.

Die erfindungsgemäßen Ester werden vorzugsweise direkt aus den Fettsäuren und pflanzenbasiertem Ethylenglykol hergestellt. Bevorzugt ist hierbei die Anwesenheit eines Katalysators, vorzugsweise Zinnoxalat in Mengen von 0,01 bis 0,5 Gew.-% - bezogen auf Ansatz. Vorteilhaft sind erhöhte Temperaturen, vorzugsweise über den Schmelztemperaturen der Fettsäuren.

Im Sinne der Erfindung sind die Ester vorzugsweise eine Mischung aus Mono- und Diester des Ethylenglykols, bevorzugt eine Mischung aus

0,5 bis 10 Gew.-%, vorzugsweise 3 bis 10 Gew.-%, Monoester des Ethylenglykols und
90 bis 99,5 Gew.-%, vorzugsweise 90 bis 97 Gew.-%, Dieester des Ethylenglykols.

Derartige Estermischungen sind erhältlich durch ein molares Verhältnis von Ethylenglykol: Carbonsäure bzw. Fettsäure von vorzugsweise 0,9 : 2 bis 1,5 : 2, insbesondere 1 : 2 bis 1,1 : 2.

Als Carbon- bzw. Fettsäuren sind beispielsweise Fettsäuren mit 6 bis 22 Kohlenstoffatomen, vorzugsweise mit 12 bis 22 Kohlenstoffatomen, wie Capronsäure, Caprylsäure, Caprinsäure, Laurinsäure, Myristinsäure, Palmitinsäure, Palmoleinsäure, Stearinsäure, Isostearinsäure, Hydroxystearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische geeignet.

Besonders geeignet sind als Ester Mono- und Diester des Ethylenglykols mit Fettsäuren mit 6 bis 22 Kohlenstoffatomen, vorzugsweise mit 12 bis 22 Kohlenstoffatomen und insbesondere Ester von Ethylenglykol mit einer Fettsäuremischung bestehend aus

40 bis 100 Gew.-% Stearinsäure und
0 bis 60 Gew.-% Palmitinsäure und
0 bis 20 Gew.-% Laurin- und/oder Myristinsäure.

Einer Ausführungsform entsprechend betrifft die Erfindung Ester von Ethylenglykol und einer Fettsäuremischung bestehend aus

85 bis 100 Gew.-% Stearinsäure und
0 bis 15 Gew.-% Palmitinsäure und
0 bis 15 Gew.-% Laurin- und/oder Myristinsäure, wobei sich die Gew.-% zu 100 addieren.

Diese Ester sind als Trübungsmittel und zur Erzeugung eines Weißgrades, vorzugsweise in Form ihrer Dispersion in kosmetischen Mitteln, geeignet.

Einer zweiten Ausführungsform entsprechend betrifft die Erfindung Ester von Ethylenglykol und einer Fettsäuremischung bestehend aus

40 bis 60 Gew.-% Stearinsäure und
40 bis 60 Gew.-% Palmitinsäure und
0 bis 20 Gew.-% Laurin- und/oder Myristinsäure, wobei sich die Gew.-% zu 100 addieren.

Innerhalb dieser zweiten Ausführungsform sind solche bevorzugt, deren Fettsäuremischung besteht aus

40 bis 60 Gew.-% Stearinsäure und
40 bis 60 Gew.-% Palmitinsäure, wobei die Gew.-% sich zu 100 addieren.

Derartige Fettsäuremischungen sind beispielsweise im Handel unter dem Handelsnamen Edenor L2SM®, von der KLK Oleo Malaysia, erhältlich.
Die davon abgeleiteten Ester sind zur Erzeugung eines Perlglanzes, vorzugsweise in Form ihrer Dispersion in kosmetischen Mitteln, geeignet.
Bevorzugt im Sinne der Erfindung sind Ester bestehend aus 3 bis 10 Gew.-% Monoester und 90 bis 97 Gew.-% Diester des Ethylenglykols von Fettsäuren bzw. einer Fettsäuremischung bestehend aus 40 bis 60 Gew.-% Stearinsäure und 40 bis 60 Gew.-% Palmitinsäure.
Bevorzugte Ethylenglykolester haben folgende Kennzahlen:

Säurezahl SZ nach DIN 53402 im Bereich von 5,5 bis 7; insbesondere 6 bis 6,5;
Hydroxylzahl OHZ nach DIN 53240 im Bereich von 5 bis 7, insbesondere 5,5 bis 6,5;
Verseifungszahl VZ nach DIN 53401 im Bereich von 185 bis 210, insbesondere von 195 bis 200.

Im Sinne der vorliegenden Erfindung sind die beschriebenen Ester wachsartig. Unter dem Begriff "Wachs" bzw. "wachsartig" wird in der vorliegenden Anmeldung eine bei Raumtemperatur feste, meist knetbare Verbindung verstanden, die ohne Zersetzung schmilzt. Der Schmelzpunkt wird im Sinne der Erfindung gemessen nach Kofler Heizbank gemäß ISO 6321 und liegt vorzugsweise über 40° C, bevorzugt unter 75° C und insbesondere zwischen 45° C und 65° C.
Ein weiterer Gegenstand der vorliegenden Erfindung sind Wachsdispersionen, die den Ester nach mindestens einem der Ansprüche 1 bis 6, vorzugsweise in Mengen von 15 bis 30 Gew.-%, bevorzugt in Mengen von 20 bis 30 Gew.-% - bezogen auf Wachsdispersion - enthalten.
Bevorzugt enthalten die erfindungsgemäßen Wachsdispersionen, zusätzlich Tenside und ggf. Konservierungsmittel.
Einer Ausführungsform entsprechend enthalten die Wachsdispersionen

**a)** 15 bis 30 Gew.-% Ester des Ethylenglykols als Wachs und
b) 10 bis 30 Gew.-% Tenside, vorzugsweise Alkylenoxid-freie Tenside
c) ggf. 0,01 bis 1,0 Gew.-% Konservierungsmittel.

Tenside

Als oberflächenaktive Stoffe (Tenside) können anionische, nichtionische, kationische, amphotere bzw. zwitterionische Tenside enthalten sein, deren Anteil in den Wachsdispersionen üblicherweise etwa 10 bis 30 Gew.-% beträgt.
Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Alk(en)ylpolyglykolethercitraten bzw. deren Salzen, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für besonders geeignete milde, d.h. besonders hautverträgliche Tenside sind Fettalkoholpolyglycolethersulfate wie Laurylethersulfat mit einem durchschnittlichen Ethoxylierungsgrad von 1 oder 2, welches beispielsweise unter dem Handelsnamen Texapon® N70 erhältlich ist, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Fettsäureglucamide und/oder Proteinfettsäurekondensate.
Typische Beispiele für kationische Tenside sind quartäre Ammoniumverbindungen, wie das Dimethyldistearylammoniumchlorid. Bevorzugt sind Ammoniumhalogenide, insbesondere Chloride und Bromide, wie Alkyltrimethylammiumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats"

werden im Allgemeinen quaternierte Fettsäuretriethanolaminestersalze verstanden. Besonders bevorzugt sind als kationische Tenside Produkte, die als Dehyquart® L80, Dehyquart® F 75, Dehyquart® A-CA bekannt sind.

Geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, $C_{12/14}$-Kokosalkyldimethylamin, Myristyldimethylamin, Cetyldimethylamin, Stearyldimethylamin, Stearylethylmethylamin, Oleyldimethylamin, $C_{16/18}$-Talgalkyldimethylamin sowie deren technische Gemische. Weiterhin kommen auch Carboxyalkylierungsprodukte von Amidoaminen in Betracht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Ganz besonders bevorzugt ist das sog. Cocoamidopropylbetain, welches unter dem Handelsnamen Dehyton® PK 45 im Handel erhältlich ist.

Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum $C_{12/14}$-Kokosfettsäure, die anschließend mit Natriumchloracetat betainisiert werden.

Typische Beispiele für nichtionische Tenside sind Fettalkohol(poly)glycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, partiell oxidierte Alk(en)yloligoglykoside bzw. Glucoronsäurederivate, Fettsäure-N-alkylglucamide, Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Beispiele für geeignete nichtionische Tenside sind ethoxylierte Fettalkohole, beispielsweise ein mit 4 Mol Ethylenoxid ethoxylierter Fettalkohol bzw. ethoxyliertes Fettalkoholgemisch mit C12/C14-Anteilen wie das im Handel erhältliche Dehydol® LS4.

Unter den nichtionischen Tensiden werden insbesondere für milde Zubereitungen Alkyl- und/oder Alkenyloligoglykoside bevorzugt, die der Formel **(II)**

$$\mathbf{RO\text{-}[G]_p} \qquad \mathbf{(II)}$$

folgen, in der R für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest R kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

Besonders bevorzugt sind als nichtionische Tenside eine Mischung von Alkylpolyglucosiden aus $C_{12}$-Alkylgucosid und $C_{8-10}$-Alkylglucosid in einem Gewichtsverhältnis von 1,5: 1 bis 2,5 :1 - bezogen auf Aktivsubstanz.

Besonders bevorzugt sind in den Wachsdispersionen als Tenside nichtionische Tenside enthalten und vorzugsweise enthalten die Wachsdispersionen

a) 15 bis 30 Gew.-% Ester des Ethylenglykols als Wachs
b) 10 bis 30 Gew.-% nichtionische Tenside, vorzugsweise ausgewählt aus der von Alkyl- und/oder Alkylenpolyglucosiden gebildeten Gruppe und
c) ggf. 0,01 bis 0,5 Gew.-% Konservierungsmittel.

Hervorragende Weißtrübungen/Perlglanz werden erhalten, wenn die Wachsdispersionen

a) 15 bis 30 Gew.-% Ester des Ethylenglykols als Wachs bestehend aus a1) 3 bis 10 Gew.-% Monoester des Ethylenglykols und a2) 90 bis 97 Gew.-% Diester des Ethylenglykols und
b) 10 bis 30 Gew.-% nichtionische Tenside ausgewählt aus der von Alkyl- und/oder Alkylenpolyglucosiden gebildeten Gruppe und
c) ggf. 0,01 bis 0,5 Gew.-% Konservierungsmittel enthalten.

Innerhalb dieser Gruppe sind Wachsdispersionen enthaltend

a) 15 bis 30 Gew.-% Ester des Ethylenglykols als Wachs bestehend aus 3 bis 10 Gew.-% Monoester und 90 bis 97 Gew.-% Diester des Ethylenglykols mit einer Mischung von Fettsäuren aus 40 bis 60 Gew.-% Stearinsäure und 40 bis 60 Gew.-% Palmitinsäure und
b) 10 bis 30 Gew.-% nichtionische Tenside ausgewählt aus der von Alkyl- und/oder Alkylenpolyglucosiden gebildeten Gruppe und
c) ggf. 0,01 bis 0,5 Gew.-% Konservierungsmittel und

insbesondere Wachsdispersionen enthaltend

a) 15 bis 30 Gew.-% Ester des Ethylenglykols als Wachs bestehend aus 3 bis 10 Gew.-% Monoester und 90 bis 97 Gew.-% Diester des Ethylenglykols mit einer Mischung von Fettsäuren aus 40 bis 60 Gew.-% Stearinsäure und 40 bis 60 Gew.-% Palmitinsäure und
b) 10 bis 30 Gew.-% einer Mischung von Alkylpolyglucosiden aus $C_{12}$-Alkylgucosid und $C_{8-10}$- Alkylglucosid in einem Gewichtsverhältnis von 1,5: 1 bis 2,5: 1- bezogen auf Aktivsubstanz - und
c) ggf. 0,01 bis 0,5 Gew.-% Konservierungsmittel

vorteilhaft.

Als ggf. enthaltene Konservierungsmittel eignen sich beispielsweise Citronensäure sowie Benzoesäure und/oder deren Salze, Phenoxyethanol, Formaldehydlösung, Parabene, Pentandiol, Sorbinsäure, Lävulinsäure und Arachidonsäure sowie die unter der Bezeichnung Surfacine® bekannten Silberkomplexe und die in Anlage 6, Teil A und B der Kosmetikverordnung aufgeführten weiteren Stoffklassen. Besonders geeignet sind Citronensäure sowie Benzoesäure und/oder deren Salze, wie Na-Salze.

Die erfindungsgemäßen Wachsdispersionen enthalten stets ad 100 Gew.-% Wasser.

Im Sinne der Erfindung enthalten die Wachsdispersionen möglichst geringe Mengen, vorzugsweise zwischen 0 und 10 Gew.-% und insbesondere keine petrochemischen Verbindungen - bezogen auf Wachsdispersion. So sollen die bevorzugten Wirkstoffe der Wachsdispersionen möglichst vollständig, vorzugsweise zwischen 90 und 100 Gew.-% und insbesondere vollständig - bezogen auf Wachsdispersion - auf nachwachsenden Rohstoffen basieren.

Ein weiterer Gegenstand der vorliegenden Erfindung ist die Verwendung der erfindungsgemäßen Ester nach mindestens einem der Ansprüche 1 bis 6 als Wachs, vorzugsweise in Form seiner erfindungsgemäßen Dispersion nach einem der Ansprüche 7 bis 13, zur Erzeugung einer Trübung oder eines Perlglanzes in wässrigen Mitteln, vorzugsweise zur Erzeugung einer Trübung oder eines Perlglanzes in kosmetischen Mitteln zur Reinigung von Haut und/oder Haar.

Vorzugsweise werden die erfindungsgemäßen Wachse in Form der erfindungsgemäßen Wachsdispersionen nach einem der Ansprüche 7 bis 13 verwendet.

Kosmetische Mittel

Ein weiterer Gegenstand der vorliegenden Erfindung betrifft kosmetische Reinigungsmittel für Haut und Haar enthaltend einen Ester des Ethylenglykols nach Anspruch 1 als Perlglanz- oder Trübungsmittel.

Unter kosmetische Mittel zur Reinigung von Haar sind Mittel zu verstehen, die wieder abgespült werden. So sind als kosmetische Mittel zur Reinigung von Haar alle kosmetischen Haarbehandlungsmittel zu verstehen, die zur alleinigen Reinigung des Haares gedacht sind als auch solche, die zur Pflege, Trocknung, Farbänderung oder Strukturänderung des Haares gedacht sind und einen Reinigungsschritt umfassen. Beispielsweise sind darunter Haarshampoos, Haarkonditionierer, konditionierende Shampoos, Haarspülungen, Haarkuren, Haarpackungen, Haar-Tonics, Dauerwell-Fixierlösungen, Haarfärbeshampoos, Haarfärbemittel oder Kombinationen davon zu verstehen.

Als kosmetische Mittel zur Reinigung von Haut sind Mittel zu verstehen, die die Hautoberfläche von Schmutz und Fett befreit und ggf. pflegt beispielsweise Duschgele, Duschbäder, Duschöle, Schaumbäder, flüssige Handseife, Intimwaschlotionen, Gesichtsreinigungslotionen.

Je nach Applikationszweck enthalten diese Zubereitungen eine Reihe weiterer Hilfs- und Zusatzstoffe wie weitere Tenside, Ölkörper, Emulgatoren, Co-Tenside, (kationische) Polymere, Konsistenzgeber, Verdickungsmittel, Überfettungsmittel, Stabilisatoren, Siliconverbindungen, Fette, Wachse, Lecithine, Phospholipide, UV-Lichtschutzfaktoren, biogene Wirkstoffe, Antioxidantien, Antischuppenmittel, Filmbildner, Quellmittel, Tyrosininhibitoren (Depigmentierungsmittel), Hydrotrope, Solubilisatoren, Konservierungsmittel, Parfümöle, Farbstoffe und dergleichen.

In den erfindungsgemäßen kosmetischen Mitteln sind die Wachse vorteilhafterweise in Form einer Dispersion vorzugsweise in Mengen von 0,1 bis 5,0, vorzugsweise in Mengen von 0,2 bis 2,5 Gew.-% und insbesondere 0,3 bis 2,0 Gew.-% - bezogen auf Gehalt an Ester des Ethylenglykols - enthalten.

Im Folgenden werden geeignete weitere Inhaltsstoffe für die erfindungsgemäße Verwendung bzw. für die erfindungsgemäßen kosmetischen Mittel aufgeführt.

Tenside

Im Sinne der vorliegenden Erfindung können die kosmetischen Mittel anionische, nichtionische, kationische sowie amphotere bzw. zwitterionische Tenside enthalten. Die Auswahl der Tenside richtet sich nach dem gewünschten Anwendungszweck. Geeignete Tenside sind bereits im Zusammenhang mit den Tensiden der erfindungsgemäßen Wachsdispersion im Rahmen dieser Anmeldung beispielshaft aufgelistet worden. Diese Beispiele sind auch als weitere Tenside für die kosmetischen Mittel geeignet. Kosmetische Mittel zur konditionierenden Haarbehandlung enthalten typischerweise neben den erfindungsgemäßen Wachsdispersionen, anionische Tenside.

Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Alk(en)ylpolyglykolethercitraten bzw. deren Salzen, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Besonders bevorzugt werden als anionische Tenside die Fettalkohol(ethersulfate), die bereits im Zusammenhang mit den erfindungsgemäßen Wachsdispersionen beschrieben wurden und insbesondere Laurylethersulfat mit 1 oder 2 Mol Ethylenoxid.

Vorzugsweise enthalten die kosmetischen Mittel zur konditionierenden Haarbehandlung neben den erfindungsgemäßen Wachsdispersionen sowie den anionischen Tensiden auch amphotere bzw. zwitterionische Tenside. Geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, $C_{12/14}$-Kokosalkyldimethylamin, Myristyldimethylamin, Cetyldimethylamin, Stearyldimethylamin, Stearylethylmethylamin, Oleyldimethylamin, $C_{16/18}$-Talgalkyldimethylamin sowie deren technische Gemische. Weiterhin kommen auch Carboxyalkylierungsprodukte von Amidoaminen in Betracht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Ganz besonders bevorzugt ist das sog. Cocoamidopropylbetain, welches unter dem Handelsnamen Dehyton® PK 45 im Handel erhältlich ist.

Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C-Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Silikonöle (cyclische Dimethicone sog. (INCI) Cyclomethicone, Polymethylsiloxane, sog. (INCI) Dimethicone, aminofunktionelle Silikone, sog. (INCI) Amodimethicone wie Trimethylsilylamodimethicone u.a.) und/ oder aliphatische bzw. naphthenische Kohlenwasserstoffe, wie z.B. Squalan, Squalen oder Dialkylcyclohexane in Betracht. Geeignete Silikonöle werden in dem Europäischen Patent EP1830798 auf den Seiten 8-14 beschrieben, auf die hier ausdrücklich Bezug genommen wird.

Emulgatoren

Als Emulgatoren kommen beispielsweise nichtionogene Tenside aus mindestens einer der folgenden Gruppen in Frage:

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Addukte mit 1 bis 30 Mol Ethylenoxid an Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen;
- Partialester von Polyglycerin (durchschnittlicher Eigenkondensationsgrad 2 bis 8), Polyethylenglycol (Molekulargewicht 400 bis 5000), Sorbitan, Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

Besonders bevorzugte Emulgatoren sind Anlagerungsprodukte von Ethylenoxid an $C_{12/18}$-Fettsäuremono- und

-diester, Anlagerungsprodukte von 1 bis 30, vorzugsweise 5 bis 10 Mol Ethylenoxid an Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische. Ebenfalls geeignet sind Anlagerungsprodukte von 1 bis 30, vorzugsweise 5 bis 10 Mol Ethylenoxid an Sorbitanester. Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische in Frage. Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen. Bevorzugt ist auch Trimethylpropan-EO/PO-Trioleat, ein Gemisch erhältlich durch die Umsetzung von Trimethylolpropantrioleat mit Ethylenoxid und Propylenoxid unter alkalischen Bedingungen. Dabei findet, zumindest zum Teil, eine Einlagerung der Ethylenoxideinheiten (EO) und der Propylenoxideinheiten (PO) in die Estergruppen des Trimethylolpropantrioleat statt. Das Trimethylpropan-EO/PO-Trioleat wird charakterisiert durch seinen Gehalt an EO- und PO-Einheiten pro Molekül im statistischen Mittel. In einer Ausführungsform der vorliegenden Erfindung wird Trimethylpropan-EO/PO-Trioleat, mit 120 Ethylenoxideinheiten (EO) und 10 Propylenoxideinheiten (PO) eingesetzt.

**Partialglyceride,** stellen Mono und/oder Diester des Glycerins mit linearen, gesättigten und/oder partiell ungesättigten Fettsäuren, beispielsweise Capronsäure, Caprylsäure, Caprinsäure, Laurinsäure, Myristinsäure, Palmitinsäure, Palmoleinsäure, Talgfettsäure, Stearinsäure, Behensäure sowie deren technische Mischungen dar. Sie folgen der Formel **(III),**

$$\begin{array}{l} CH_2O(CH_2CH_2O)_x\text{-}COR^5 \\ \quad | \\ CH\text{-}O(CH_2CH_2O)_yR^6 \\ \quad | \\ CH_2O(CH_2CH_2O)_z\text{-}R^7 \end{array} \qquad \textbf{(III)}$$

in der $R^5CO$ für einen Acylrest mit 6 bis 22 Kohlenstoffatomen, vorzugsweise für einen linearen, gesättigten Acylrest mit 6 bis 22 Kohlenstoffatomen, $R^6$ und $R^7$ unabhängig voneinander für Wasserstoff oder $R^5CO$, x, y und z in Summe für 0 oder für Zahlen von 1 bis 30 und X für ein Alkali- oder Erdalkalimetall mit der Maßgabe steht, dass mindestens einer der beiden Reste $R^6$ und $R^7$ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Talgfettsäuremonoglycerid, Talgfettsäurediglycerid, Behensäuremonoglycerid, Behensäurediglycerid sowie deren technische Gemische, die untergeordnet aus dem Herstellungsprozess noch geringe Mengen an Triglycerid enthalten können.

Als Wachskörper kommen weiterhin als bevorzugte Gruppe **Ester von mehrwertigen, gegebenenfalls hydroxysubstituierten Carbonsäuren** mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll- oder Partialester vorliegen, vorzugsweise werden Mono- und vor

allem Diester der Carbon- bzw. Hydroxycarbonsäuren eingesetzt. Typische Beispiele sind Bernsteinsäuremono- und -dilaurylester, Bernsteinsäuremono- und -dicetearlyester, Bernsteinsäuremono- und -distearylester, Weinsäuremono- und -dilaurylester, Weinsäuremono- und -dikokosalkylester, Weinsäuremono- und -dicetearylester, Citronensäuremono-, -di- und -trilaurylester, Citronensäuremono-, -di- und -trikokosalkylester sowie Citronensäuremono-, -di- und - tricetearylester.

Als dritte bevorzugte Gruppe von Wachskörpern können **Fettalkohole** eingesetzt werden, die der Formel **(IV)** folgen,

$$R^8OH \qquad (IV)$$

in der $R^8$ für einen linearen, gegebenenfalls hydroxysubstituierten Alkylrest und/oder Acylrest mit 16 bis 48, vorzugsweise 18 bis 36 Kohlenstoffatomen steht. Typische Beispiele für geeignete Alkohole sind Cetearylalkohol, Hydroxystearylalkohol, Behenylalkohol sowie Oxidationsprodukte langkettiger Paraffine.

**Fettketone,** die als Komponente in Betracht kommen, folgen vorzugsweise der Formel **(V),**

$$R^9\text{-CO-}R^{10} \qquad (V)$$

in der $R^9$ und $R^{10}$ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste $R^{13}$ und $R^{14}$ aber nur um ein Kohlenstoffatom und leiten sich von Fettsäuren mit 16 bis 22 Kohlenstoffatomen ab.

Als Wachskörper geeignete **Fettaldehyde** entsprechen vorzugsweise der Formel **(VI),**

$$R^{11}COH \qquad (VI)$$

in der $R^{11}CO$ für einen linearen oder verzweigten Acylrest mit 24 bis 48, vorzugsweise 28 bis 32 Kohlenstoffatomen steht.

Ebenso kommen **Fettether** vorzugsweise der Formel **(VII)** in Frage,

$$R^{12}\text{-O-}R^{13} \qquad (VII)$$

in der $R^{12}$ und $R^{13}$ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.

Als Komponente kommen weiterhin **Fettcarbonate** vorzugsweise der Formel **(VIII)** in Betracht,

$$R^{14}O\text{-CO-}OR^{15} \qquad (VIII)$$

in der $R^{14}$ und $R^{15}$ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Die Stoffe werden erhalten, indem man beispielsweise Dimethyl- oder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen $R^{14}$ und $R^{15}$ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/oder Erucylalkohol in Form ihrer Mono- und Diester bzw. deren technischen Mischungen.

Bei den **Epoxidringöffnungsprodukten** handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel **(IX),**

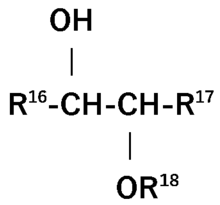

(IX)

in der $R^{16}$ und $R^{17}$ für Wasserstoff oder einen Alkylrest mit 10 bis 20 Kohlenstoffatomen steht, mit der Maßgabe, dass die Summe der Kohlenstoffatome von $R^{16}$ und $R^{17}$ im Bereich von 10 bis 20 liegt und $R^{18}$ für einen Alkyl- und/oder Alkenylrest mit 12 bis 22 Kohlenstoffatomen und/oder den Rest eines Polyols mit 2 bis 15 Kohlenstoffatomen und 2 bis 10 Hydroxylgruppen steht. Typische Beispiele sind Ringöffnungsprodukte von α-Dodecenepoxid, α-Hexadecenepoxid, α-Octadecenepoxid, α-Eicosenepoxid, α-Docosenepoxid, i-Dodecenepoxid, i-Hexadecenepoxid, i-Octadecenepoxid, i-Eicosenepoxid und/ oder i-Docosenepoxid mit Laurylalkohol, Kokosfettalkohol, Myristylalkohol, Cetylalkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin.

Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Stabilisatoren

Als Stabilisatoren können Metallsalze von Fettsäuren, wie z.B. Magnesium-, Aluminium- und/oder Zinkstearat bzw. -ricinoleat eingesetzt werden.

(kationische) Polymere

Vorzugsweise enthalten die kosmetischen Mittel zur konditionierenden Haarbehandlung neben den erfindungsgemäßen Wachsdispersionen, den anionischen Tensiden und ggf. amphoteren bzw. zwitterionischen Tensiden auch kationische Polymere. Geeignete kationische Polymere sind vorzugsweise solche aus der Gruppe der kationisch modifizierten Cellulosederivate, PQ 10, PQ 67, kationisch modifizierten Guarderivate wie Dehyquart® Guar N, Guar Hydroxypropyltrimonium Chlorid, kationischen Homo- oder Copolymeren auf der Basis von Acrylamid, kationischen Homo- oder Copolymeren auf der Basis von Vinylpyrrolidon, kationischen Homo- oder Copolymeren auf der Basis von quaternisiertem Vinylimidazol und kationischen Homo- oder Copolymeren auf der Basis von Methacrylaten.

Geeignete kationische Polymere sind beispielsweise die quaternierte Hydroxyethylcellulose, die auch unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Proteinhydrolysate, Polypeptide und Aminsäuren wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol. Insbesondere geeignete kationische Polymere sind Polyquaternium-68, erhältlich als Luviquat® Supreme AT 1, oder Polyquaternium-11, erhältlich als Luviquat® PQ 11 AT 1.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butyl maleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere,Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultra-violette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-metho-xydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenyl-zimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

<u>Biogene Wirkstoffe und Antioxidantien</u>

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, $\beta$- Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt oder Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether,

Zucker, Nukleotide, Nukleoside, Peptide und Lipide) dieser genannten Wirkstoffe.

Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Antischuppenwirkstoffe

Als Antischuppenwirkstoffe kommen Piroctón Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Ketoconazol, Elubiol, Selendisulfid, Schwefelkolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

Weitere Zusatzstoffe

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden. Als Insekten-Repellentien kommen N,N-Diethyl-m-toluamid, 1,2-Pentandiol oder Ethyl Butylacetyl-aminopropionate in Frage, als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Betracht.

Proteinhydrolysate

Falls gewünscht können weitere aus dem Stand der Technik bekannte Proteinhydrolysate eingesetzt werden, beispielsweise auf Basis von Keratin wie das im Handel erhältliche Nutrilan® Keratin W PP oder auf Basis von Weizen wie Gluadin® WLM Benz, Gluadin® WK oder Gluadin® WP. Es ist auch möglich, geringe Mengen an freien Aminosäuren wie Lysin oder Arginin zuzugeben.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10, wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Konservierungsmittel

Als Konservierungsmittel eignen sich beispielsweise Benzoate, Phenoxyethanol, Formaldehydlösung, Parabene, Pentandiol, Sorbinsäure, Lävulinsäure und Arachidonsäure sowie die unter der Bezeichnung Surfacine® bekannten Silberkomplexe und die in Anlage 6, Teil A und B der Kosmetikverordnung aufgeführten weiteren Stoffklassen.

Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, $\alpha$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Pigmente

Die erfindungsgemäßen Ester können insbesondere in Form ihrer Wachsdispersionen eine hervorragende Weißtrübung in den kosmetischen Mitteln erzeugen. Falls man den Perlglanz noch verstärken möchte, ist es möglich Pigmente zuzugeben.

Der Begriff Pigment umfasst Partikel jeder Form, die weiß oder gefärbt, organisch oder anorganisch sind, in den Zubereitungen nicht löslich sind und dem Zweck dienen, der Zubereitung einen erhöhten Glanz zu geben.

Vorteilhaft sind die Glanzpigmente, zu denen nach DIN 55944: 2003-11 die Metalleffektpigmente und die Perlglanzpigmente gehören.

Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelsübliche Effektpigmente sind: Timiron® and #174; von Merck,

Iriodin® and #174; von Merck (Perl- und Farbglanzpigmente für dekorative technische Anwendungen), Xirallic® and #174; von Merck (farbintensive Kristalleffektpigmente).

Die Pigmente können auch in Form kommerziell erhältlicher öliger oder wässriger Vordispersionen zur Anwendung kommen.

Kosmetische Haarbehandlungsmittel

Gemäß einer besonderen Ausführungsform der vorliegenden Erfindung werden kosmetische Mittel zur konditionierenden Haarbehandlung beansprucht, die die erfindungsgemäßen Wachsdispersionen, anionische Tenside und optional kationische Polymere sowie optional amphotere und/oder zwitterionische Tenside und Emulgatoren oder weitere übliche Inhaltsstoffe enthalten sowie zur Ergänzung auf 100 Gew.-% Wasser.

Vorzugsweise sind in den konditionierenden Haarbehandlungsmittel - bezogen auf Aktivsubstanzgehalt - enthalten

0,1 bis 5,0 Gew.-%, vorzugsweise 0,2 bis 2,5 Gew.-%, erfindungsgemäße Wachsdispersion,
1,0 bis 15 Gew.-%, vorzugsweise 7,5 bis 12 Gew.-%, anionische Tenside,
0,0 bis 1 Gew.-%, vorzugsweise 0,05 bis 0,4 Gew.-%, mindestens eines kationisch modifizierten Polymers und/oder
0,0 bis 15 Gew.-%, vorzugsweise 0,1 bis 5 Gew.-%, amphotere und/oder zwitterionische Tenside,
0,0 bis 10 Gew.-% Emulgatoren und optional weitere übliche Inhaltsstoffe enthalten sein können und zur Ergänzung auf 100 Gew.-% Wasser.

Die Wachsdispersion, anionische Tenside, sowie die optional enthaltenen kationischen Polymere sowie amphotere und/oder zwitterionische Tenside sowie die optional weiteren üblichen Inhaltsstoffe sind bereits oben in dieser Anmeldung beschrieben worden.

Verfahren

Weitere Gegenstände der vorliegenden Erfindung betreffen ein Verfahren zur Herstellung der erfindungsgemäßen kosmetischen Reinigungsmittel. Nach einem der erfindungsgemäßen Verfahren werden die erfindungsgemäßen wachsartigen Ester des Ethylenglykols in Form der Wachsdispersion vorzugsweise zu einem oder mehreren, vorgelegten Bestandteilen der kosmetischen Mittel bei Raumtemperatur, vorzugsweise zwischen 15 und 30° C, zugegeben und insbesondere unter Einsatz eines handelsüblichen Rührers verrührt, sog. Kaltverfahren.

Nach einer weiteren Verfahrensvariante können die kosmetischen Mittel hergestellt werden, indem die erfindungsgemäßen Ester des Ethylenglykols auf Temperaturen oberhalb ihres Schmelzpunktes erhitzt und mit mindestens einem der restlichen Bestandteile der kosmetischen Mittel verrührt werden. Vorzugsweise wird der wachsartige Ester des Ethylenglykols zusammen mit einem weiteren bei Raumtemperatur festen Bestandteil des kosmetischen Mittels auf Temperaturen oberhalb des Schmelzpunktes des erfindungsgemäßen Esters erhitzt und verrührt, bevor anschließend die weiteren, restlichen Bestandteile des kosmetischen Mittels zugegeben und verrührt werden.

## Beispiele

### A) Herstellbeispiele für erfindungsgemäßes Ethylenglykoldistearat

#### Erfindungsgemäßes Beispiel A1

Eine Mischung aus 2172,5 g (= 35 mol) pflanzlich basiertem Ethylenglykol (Kenndaten: mind. 99,8 Gew.-% Monoethylenglykol und max. 0,06 Diethylenglykol; max.0,06 Gew.-% Wasser; im Handel erhältlich von der India Glycols Bio MEG) und 19913,6 g (= 70 mol) technischer Stearinsäure (Zusammensetzung $C_{16}$-Fettsäure : $C_{18}$-Fettsäure = 1 : 1; im Handel erhältlich als Palmera B1804® der Fa. KLK Oleo) sowie 11 g Zinnoxalat (Veresterungskatalysator im Handel erhältlich als Fascat® 2001 von PMC Organometallix, Inc.) wurde auf 160° C erwärmt. Innerhalb von 16 Stunden wurde in etwa 80 % der theoretischen Wassermenge abdestilliert. Anschließend wurde der Ansatz auf 240° C erwärmt und die Restmenge Wasser innerhalb von 26 Stunden abgetrennt.

Zur Aufarbeitung wurde das Produkt mit einer NaOH-Lösung (184,6 g gelöst in 500 g vollentionisiertes Wasser) 30 Minuten bei 80° C gerührt, filtriert und getrocknet.

Man erhielt 14998 g eines weißen, festen Produktes mit folgenden Kennzahlen:

Säurezahl SZ nach DIN 53402 = 1,6
Hydroxylzahl OHZ nach DIN 53240 = 4,7

Verseifungszahl VZ nach DIN 53401= 196,2
Schmelzpunkt nach Kofler Heizbank = 63,2° C

**Vergleichsbeispiel Ethylenglykoldistearat A2 (nicht erfindungsgemäß)**

Als Vergleich diente Ethylenglykoldistearat hergestellt aus petrochemischem Ethylenglykol (Kenndaten mind. 99,9 Gew.-% Monoethylenglykol und max. 0,05 Diethylenglykol; max. 0,05 Gew.-% Wasser) mit folgenden Kennzahlen:

Säurezahl SZ nach DIN 53402 <1
Hydroxylzahl OHZ nach DIN 53240 <15
Schmelzpunkt nach Kofler Heizbank= 63,2° C
beispielsweise auf dem Markt erhältlich als Cutina® AGS, BASF Personal Care & Nutrition GmbH.

Die derzeitge 100% biogene Aktivität, d.h. biogener Anteil an $^{14}C$ Kohlenstoff in % ist für 2018 gemäß DIN mit 13,6 dpm/gC matrixunabhängig vorgegeben.
Das nach Beispiel 1 hergestellte erfindungsgemäße Ethylenglykoldistearat sowie das petrochemische Ethylenglykoldistearat gemäß Vergleichsbeispiel zeigen folgende $^{14}C$-Gehalte (8 Messungen):

| | % $^{14}C$ | | C-Kettenverteilung $C_{16}$ und $C_{18}$ | GC Gew.-% | |
|---|---|---|---|---|---|
| | | % $^{14}C$ | C16 C18 | Monoester | Diester |
| Erfindungsgemäß nach A1 | | 100 | 44,4: 52,5 | 0,9 | 98,8 |
| Vergleich A2 | | 98 | 48-52: 48-52 | 8 | 92 |
| Ethylenglykol als Basis für A1 | | 100 | Nicht vorhanden, da kein Ester | - | - |

**B) Herstellung der Wachsdispersionen/Perlglanzkonzentrate**

Es wurden folgende Perlglanzkonzentrate bzw. Wachsdispersionen hergestellt (Tabelle 1). Die Mengenangaben sind in Gew.-% Aktivsubstanz AS - bezogen auf Wachsdispersion bzw. Perlglanzkonzentrat - angegeben.
Zur Herstellung der Wachsdispersion wurde 2/3 des gesamten Wassers vorgelegt und auf 85 ° C erhitzt. In die Heißphase wurden nacheinander Ethylenglykoldistearat (erfindungsgemäß bzw. Vergleich) sowie die Alkylpolyglucoside eingerührt. Bei 64° C wurde das letzte 1/3 des gesamten Wassers zugegeben und gerührt. Ab 40° C wurden Na-Benzoat, Na-Sulfat sowie Zitronensäure zugegeben und nachgerührt.

**Tabelle 1: Wachsdispersionen**

| **Zusammensetzung (INCI)** | **B1** | **Vergleich B2** |
|---|---|---|
| **Wachskörper**<br>erfindungsgemäßes Ethylenglykoldistearat nach A1 | 22,5 | - |
| **Wachskörper**<br>Ethylenglykoldistearat gemäß Vergleich A2 | - | 22,5 |
| Lauryl Glucoside* Plantacare 1200® | 19 | 19 |
| C8/C10-Alkyl Glucoside* Glucopon 215 UP® | 8,3 | 8,3 |
| Benzoesäure | 0,5 | 0,5 |
| Zitronensäure 50%ig | 12,4 | 12,4 |
| Wasser | Ad100 | Ad100 |
| pH | 4,7 | 4,7 |
| Viskosität | 8800 | 12360 |
| Viskosität nach Nachverdickung bei 40° C | 12120 | 18200 |
| Aussehen Weißgrad | + | 0 |

(fortgesetzt)

| | | |
|---|---|---|
| **Wachskörper**<br>Aussehen Perlglanz | + | 0 |
| *Mengenangabe bezogen auf Aktivsubstanz | | |

Die Viskosität wurde mit der Brookfieldmethode (23° C, Spindel 5, 10 Upm, mPas) bestimmt. Der Weißgrad/Perlglanz wurde mit dem bloßen Auge optisch im Vergleich miteinander ermittelt.

Wie aus Tabelle 1 ersichtlich, zeigt die Wachsdispersion mit dem erfindungsgemäßen pflanzenbasierten Ethylenglykoldifettsäureester A1 einen besseren Weißgrad/Perlglanz und eine niedrigere Viskosität im Vergleich zu der Wachsdispersion mit dem petrochemisch basierten Ethylenglykoldistearat A2.

**C) Herstellung weiterer Wachsdispersionen/Perlglanzkonzentrate**

Es wurden weitere Perlglanzkonzentrate bzw. Wachsdispersionen gemäß Tabelle 2) hergestellt. Die Mengenangaben sind in Gew.-% Aktivsubstanz AS - bezogen auf Wachsdispersion bzw. Perlglanzkonzentrat - angegeben.

Zur Herstellung der Wachsdispersion wurde Wasser, Ethylenglykoldistearat (erfindungsgemäß) sowie die Tenside Laureth-4 (mit durchschnittlich 4 Mol Ethylenoxid ethoxylierter C12-Fettalkohol), Cocoamidompropylbetain und Sodium Laureth Sulfat (Na-Salz eines mit durchschnittlich 1 Ethylenoxid ethoxyliertem C12-Ethersulfates) und Na-Benzoat unter Rühren bei etwa 50rpm auf 85° C erwärmt. Man lässt die Dispersion bei unter Rühren bei etwa 50rpm auf 25° C abkühlen. Dann wurde Zitronensäure zugegeben und nachgerührt.

**Tabelle 2: Wachsdispersionen C1-C4**

| Zusammensetzung (INCI) | C1 | C2 | C3 | C4 |
|---|---|---|---|---|
| **Wachskörper**<br>erfindungsgemäßes Ethylenglykoldistearat nach A1 | 20 | 26 | 18 | 20 |
| **Tensid**<br>Laureth-4 | 5 | 12 | - | - |
| **Tensid**<br>Cocoamidopropylbetain | - | 8 | 2 | 20 |
| **Tensid**<br>Sodium Laureth-1 Sulfat | - | - | 20 | - |
| Na-Benzoat | 0.5 | 0.5 | 0.5 | 0.5 |
| Zitronensäure 50%ig | 0,8 | 0.8 | 0.8 | 0.8 |
| Wasser | Ad100 | Ad100 | | |
| pH (10%ig in Wasser) | 4,5 | 4,2 | 4,4 | 4,2 |
| Viskosität in mPas;Brookfield RVT,Spindel 4/10rpm | 2.500 | 8.000 | 3.500 | 3.000 |
| Aussehen Weißgrad | + | + | + | + |
| Aussehen Perlglanz | + | + | + | + |

**D) Herstellung eines Haarshampoos mit der Wachsdispersion B1)**

Zur Herstellung des Haarshampoos wurde bei Raumtemperatur gemäß Tabelle 3) Wasser vorgelegt und Guarhydroxypropyltrimmonium Chlorid (Verdicker) eingestreut und dispergiert. Hierin wurden die Tenside Laureth-4 (mit durchschnittlich 4 Mol Ethylenoxid ethoxylierter C12-Fettalkohol) und Cocoamidompropylbetain gelöst. Danach wurde die Wachsdispersion aus Beispiel B1) sowie die weiteren Bestandteile zugegeben und verrührt. Die Mengenangaben in Tabelle 3) sind in Gew.-%, wobei die Gew.-% sich bei der Wachsdispersion auf die Wachsdispersion nach B1) und bei den Tensiden sich die Gew.-% auf den Aktivsubstanzgehalt AS - bezieht.

**Tabelle 3: Haarshampoo D1)**

| **Zusammensetzung (INCI)** | **D1** |
|---|---|
| Wasser | 82.5 |
| **Tensid**<br>Sodium Laureth-1 Sulfat | 10.0 |
| **Tensid**<br>Cocoamidopropylbetain | 2.0 |
| Guarhydroxypropyltrimmonium Chlorid | 0.2 |
| Wachsdispersion B1) | 2 |
| Na-Benzoat | 0.5 |
| Zitronensäure 50%ig | 0.3 |
| Natriumchlorid | 2.0 |
| Parfum | 0.3 |
| pH (10%ig in Wasser) | 4,8 |
| Viskosität in mPas;Brookfield RVT,Spindel 4/10rpm | 10.000 |
| Aussehen Weißgrad | + |
| Aussehen Perlglanz | + |

**Patentansprüche**

1. Wachsartige Ester des Ethylenglykols, insbesondere geeignet als Trübungs- und/oder Perlglanzmittel in kosmetischen Reinigungsmitteln, **dadurch gekennzeichnet, dass** das Ethylenglykol im Ester nach der $^{14}C$-Analytik mindestens 99% biogenen Anteil an Kohlenstoff aufweist, der gemäß DIN EN 15440 mit 13,6 dpm/gC matrixunabhängig vorgegeben ist; mit einer Standardabweichung von +/- 0 bis +/- 4.

2. Wachsartige Ester des Ethylenglykols nach Anspruch 1, **dadurch gekennzeichnet, dass** das Ethylenglykol im Ester nach der $^{14}C$-Analytik 100% biogenen Anteil an Kohlenstoff aufweist, der gemäß DIN EN 15440 mit 13,6 dpm/gC matrixunabhängig vorgegeben ist; mit einer Standardabweichung von +/- 0 bis +/- 4.

3. Wachsartige Ester des Ethylenglykols nach einem der Ansprüche 1 oder 2, **dadurch gekennzeichnet, dass** sie eine Mischung aus Mono- und Diester des Ethylenglykols enthalten, vorzugsweise eine Mischung aus

   0,5 bis 10 Gew.-% Monoester des Ethylenglykols und
   90 bis 99,5 Gew.-% Dieester des Ethylenglykols.

4. Wachsartige Ester des Ethylenglykols nach einem der Ansprüche 1 bis 3, **dadurch gekennzeichnet, dass** sie Mono- und Diester des Ethylenglykols mit Fettsäuren mit 6 bis 22 Kohlenstoffatomen, vorzugsweise Ester des Ethylenglykols mit Fettsäuren mit 12 bis 22 Kohlenstoffatomen sind.

5. Wachsartige Ester des Ethylenglykols nach einem der Ansprüche 1 bis 4, **dadurch gekennzeichnet, dass** sie Mono- und Diester des Ethylenglykols mit Fettsäuremischungen bestehend aus

   40 bis 100 Gew.-% Stearinsäure und
   0 bis 60 Gew.-% Palmitinsäure und
   0 bis 20 Gew.-% Laurin- und/oder Myristinsäure, enthalten.

6. Wachsartige Ester des Ethylenglykols nach einem der Ansprüche 1 bis 5, **dadurch gekennzeichnet, dass** sie bestehen aus 3 bis 10 Gew.-% Monoester und 90 bis 97 Gew.-% Diester des Ethylenglykols von einer Mischung von Fettsäuren bestehend aus 40 bis 60 Gew.-% Stearinsäure und 40 bis 60 Gew.-% Palmitinsäure.

7. Wachsdispersionen **dadurch gekennzeichnet, dass** sie die wachsartigen Ester des Ethylenglykols nach mindestens einem der Ansprüche 1 bis 6 in Mengen von 15 bis 30 Gew.-% - bezogen auf Wachsdispersion - enthalten.

8. Wachsdispersionen nach Anspruch 7, **dadurch gekennzeichnet, dass** sie

   a) 15 bis 30 Gew.-% Ester des Ethylenglykols als Wachs und
   b) 10 bis 30 Gew.-% Tenside, vorzugsweise Alkylenoxid-freie Tenside und
   c) ggf. 0,01 bis 1,0 Gew.-% Konservierungsmittel enthalten.

9. Wachsdispersionen nach einem der Ansprüche 7 oder 8, **dadurch gekennzeichnet, dass** sie

   a) 15 bis 30 Gew.-% Ester des Ethylenglykols als Wachs
   b) 10 bis 30 Gew.-% nichtionische Tenside, vorzugsweise ausgewählt aus der von Alkyl- und/oder Alkylenpolyglucosiden gebildeten Gruppe und
   c) ggf. 0,01 bis 0,5 Gew.-% Konservierungsmittel enthalten.

10. Wachsdispersionen nach einem der Ansprüche 7 bis 9, **dadurch gekennzeichnet, dass** sie

   a) 15 bis 30 Gew.-% Ester des Ethylenglykols als Wachs bestehend aus a1) 3 bis 10 Gew.-% Monoester des Ethylenglykols und a2) 90 bis 97 Gew.-% Diester des Ethylenglykols und
   b) 10 bis 30 Gew.-% nichtionische Tenside ausgewählt aus der von Alkyl- und/oder Alkylenpolyglucosiden gebildeten Gruppe und
   c) ggf. 0,01 bis 0,5 Gew.-% Konservierungsmittel enthalten.

11. Wachsdispersionen nach einem der Ansprüche 7 bis 10, **dadurch gekennzeichnet, dass** sie

   a) 15 bis 30 Gew.-% Ester des Ethylenglykols als Wachs bestehend aus 3 bis 10 Gew.-% Monoester und 90 bis 97 Gew.-% Diester des Ethylenglykols mit einer Mischung von Fettsäuren aus 40 bis 60 Gew.-% Stearinsäure und 40 bis 60 Gew.-% Palmitinsäure und
   b) 10 bis 30 Gew.-% nichtionische Tenside ausgewählt aus der von Alkyl- und/oder Alkylenpolyglucosiden gebildeten Gruppe und
   c) ggf. 0,01 bis 0,5 Gew.-% Konservierungsmittel enthalten.

12. Wachsdispersionen nach einem der Ansprüche 7 bis 11, **dadurch gekennzeichnet, dass** sie keine petrochemischen Verbindungen enthalten.

13. Wachsdispersionen nach einem der Ansprüche 7 bis 12, **dadurch gekennzeichnet, dass** die Ester des Ethylenglykols einen Schmelzpunkt über 40° C, vorzugsweise unter 75° C und insbesondere zwischen 45 und 65° C aufweisen.

14. Verwendung der Ester nach mindestens einem der Ansprüche 1 bis 6 als Wachs, vorzugsweise in Form seiner Dispersion nach einem der Ansprüche 7 bis 13 zur Erzeugung einer Trübung oder eines Perlglanzes, vorzugsweise zur Erzeugung einer Trübung oder eines Perlglanzes in kosmetischen Mitteln zur Reinigung von Haut und/oder Haar.

15. Verwendung nach Anspruch 14, **dadurch gekennzeichnet, dass** die Wachse in Form von Wachsdispersionen nach einem der Ansprüche 7 bis 13 verwendet werden.

16. Kosmetische Reinigungsmittel für Haut und Haar enthaltend einen Ester des Ethylenglykols nach Anspruch 1 als Perlglanz- oder Trübungsmittel.

17. Verfahren zur Herstellung von kosmetischen Reinigungsmitteln nach Anspruch 16, **dadurch gekennzeichnet, dass** die Ester des Ethylenglykols nach Anspruch 1 in Form von Dispersionen nach mindestens einem der Ansprüche 7 bis 13 bei Raumtemperatur zu einem oder mehreren, vorgelegten Bestandteilen des kosmetischen Mittels, vorzugsweise zwischen 15 und 30° C, zugegeben verrührt werden.

18. Verfahren zur Herstellung von kosmetischen Reinigungsmitteln nach Anspruch 16, **dadurch gekennzeichnet, dass** die Ester des Ethylenglykols nach Anspruch 1 auf Temperaturen oberhalb des Schmelzpunktes erhitzt werden, und mit mindestens einem der restlichen Bestandteile des kosmetischen Mittels verrührt werden.

**Claims**

1. Waxy esters of ethylene glycol, in particular suitable as an opacifying and/or pearlescent agent in cosmetic cleansers, wherein the ethylene glycol in the ester according to $^{14}C$ analysis has an at least 99% biogenic proportion of carbon, defined according to DIN EN 15440 at 13.6 dpm/gC independently of the matrix, with a standard deviation of +/- 0 to +/- 4.

2. The waxy esters of ethylene glycol according to claim 1, wherein the ethylene glycol in the ester according to $^{14}C$ analysis has a 100% biogenic proportion of carbon, defined according to DIN EN 15440 at 13.6 dpm/gC independently of the matrix, with a standard deviation of +/- 0 to +/- 4.

3. The waxy esters of ethylene glycol according to either of claims 1 and 2, wherein they comprise a mixture of monoester and diester of ethylene glycol, preferably a mixture of
0.5% to 10% by weight of monoester of ethylene glycol and 90% to 99.5% by weight of diester of ethylene glycol.

4. The waxy esters of ethylene glycol according to any of claims 1 to 3, wherein they are mono- or diesters of ethylene glycol with fatty acids having 6 to 22 carbon atoms, preferably esters of ethylene glycol with fatty acids having 12 to 22 carbon atoms.

5. The waxy esters of ethylene glycol according to any of claims 1 to 4, wherein they comprise mono- and diesters of ethylene glycol with fatty acid mixtures consisting of

   40% to 100% by weight of stearic acid and
   0% to 60% by weight of palmitic acid and
   0% to 20% by weight of lauric and/or myristic acid.

6. The waxy esters of ethylene glycol according to any of claims 1 to 5, wherein they consist of 3% to 10% by weight of monoester and 90% to 97% by weight of diester of ethylene glycol of a mixture of fatty acids consisting of 40% to 60% by weight of stearic acid and 40% to 60% by weight of palmitic acid.

7. A wax dispersion, wherein it comprises the waxy esters of ethylene glycol according to at least one of claims 1 to 6 in amounts of 15% to 30% by weight, based on the wax dispersion.

8. The wax dispersion according to claim 7, wherein it comprises

   a) 15% to 30% by weight of esters of ethylene glycol as wax and
   b) 10% to 30% by weight of surfactants, preferably alkylene oxide-free surfactants, and
   c) optionally 0.01% to 1.0% by weight of preservatives.

9. The wax dispersion according to either of claims 7 and 8, wherein it comprises

   a) 15% to 30% by weight of esters of ethylene glycol as wax,
   b) 10% to 30% by weight of nonionic surfactants, preferably selected from the group made up of alkyl and/or alkylene polyglucosides, and
   c) optionally 0.01% to 0.5% by weight of preservatives.

10. The wax dispersion according to any of claims 7 to 9, wherein it comprises

   a) 15% to 30% by weight of esters of ethylene glycol as wax consisting of a1) 3% to 10% by weight of monoester of ethylene glycol and a2) 90% to 97% by weight of diester of ethylene glycol, and
   b) 10% to 30% by weight of nonionic surfactants selected from the group made up of alkyl and/or alkylene polyglucosides, and
   c) optionally 0.01% to 0.5% by weight of preservatives.

11. The wax dispersion according to any of claims 7 to 10, wherein it comprises

   a) 15% to 30% by weight of esters of ethylene glycol as wax consisting of 3% to 10% by weight of monoester and 90% to 97% by weight of diester of ethylene glycol with a mixture of fatty acids composed of 40% to 60%

by weight of stearic acid and 40% to 60% by weight of palmitic acid and
b) 10% to 30% by weight of nonionic surfactants selected from the group made up of alkyl and/or alkylene polyglucosides, and
c) optionally 0.01% to 0.5% by weight of preservatives.

**12.** The wax dispersion according to any of claims 7 to 11, wherein it does not comprise any petrochemical compounds.

**13.** The wax dispersion according to any of claims 7 to 12, wherein the esters of ethylene glycol have a melting point above 40°C, preferably below 75°C and in particular between 45 and 65°C.

**14.** The use of the esters according to at least one of claims 1 to 6 as wax, preferably in the form of its dispersion according to any of claims 7 to 13, for generating cloudiness or pearlescence, preferably for generating cloudiness or pearlescence in cosmetic compositions for cleansing skin and/or hair.

**15.** The use according to claim 14, wherein the waxes are used in the form of a wax dispersion according to any of claims 7 to 13.

**16.** A cosmetic cleanser for skin and hair comprising an ester of ethylene glycol according to claim 1 as a pearlescent or opacifying agent.

**17.** A process for the production of a cosmetic cleanser according to claim 16, wherein the esters of ethylene glycol according to claim 1 in the form of a dispersion according to at least one of claims 7 to 13 at room temperature are added with stirring to one or more initially charged constituents of the cosmetic composition, preferably between 15 and 30°C.

**18.** A process for the production of a cosmetic cleanser according to claim 16, wherein the esters of ethylene glycol according to claim 1 are heated to temperatures above the melting point and are stirred with at least one of the remaining constituents of the cosmetic composition.

**Revendications**

**1.** Esters cireux de l'éthylène glycol, en particulier appropriés en tant qu'agent opacifiant et/ou agent nacrant dans des agents de nettoyage cosmétiques, **caractérisés en ce que** l'éthylène glycol dans l'ester présente, d'après une analyse du $^{14}C$, au moins 99 % de part de carbone biogène, qui est prédéfini indépendamment de la matrice selon la norme DIN EN 15440 avec 13,6 dpm/gC ; avec un écart type de $\pm$ 0 à $\pm$ 4.

**2.** Esters cireux de l'éthylène glycol selon la revendication 1, **caractérisés en ce que** l'éthylène glycol dans l'ester présente, d'après une analyse du $^{14}C$, 100 % de part de carbone biogène, qui est prédéfini indépendamment de la matrice selon la norme DIN EN 15440 avec 13,6 dpm/gC ; avec un écart type de $\pm$ 0 à $\pm$ 4.

**3.** Esters cireux de l'éthylène glycol selon l'une quelconque des revendications 1 ou 2, **caractérisés en ce qu'**ils contiennent un mélange de monoester et de diester de l'éthylène glycol, de préférence un mélange composé de

0,5 à 10 % en poids de monoester de l'éthylène glycol et
90 à 99,5 % en poids de diester de l'éthylène glycol.

**4.** Esters cireux de l'éthylène glycol selon l'une quelconque des revendications 1 à 3, **caractérisés en ce qu'**ils sont des monoesters et des diesters de l'éthylène glycol avec des acides gras comportant 6 à 22 atomes de carbone, de préférence des esters de l'éthylène glycol avec des acides gras comportant 12 à 22 atomes de carbone.

**5.** Esters cireux de l'éthylène glycol selon l'une quelconque des revendications 1 à 4, **caractérisés en ce qu'**ils contiennent des monoesters et des diesters de l'éthylène glycol avec des mélanges d'acides gras constitués de

40 à 100 % en poids de l'acide stéarique et
0 à 60 % en poids d'acide palmitique et
0 à 20 % en poids d'acide laurique et/ou myristique.

6. Esters cireux de l'éthylène glycol selon l'une quelconque des revendications 1 à 5, **caractérisés en ce qu'**il sont constitués de 3 à 10 % en poids de monoester et de 90 à 97 % en poids de diester de l'éthylène glycol d'un mélange d'acides gras constitués de 40 à 60 % en poids d'acide stéarique et de 40 à 60 % en poids d'acide palmitique.

7. Dispersions de cire **caractérisées en ce qu'**elles contiennent les esters cireux de l'éthylène glycol selon au moins l'une des revendications 1 à 6 en des quantités de 15 à 30 % en poids - par rapport à la dispersion de cire.

8. Dispersions de cire selon la revendication 7, **caractérisées en ce qu'**elles contiennent

a) 15 à 30 % en poids d'ester de l'éthylène glycol en tant que cire et
b) 10 à 30 % en poids de tensioactifs, de préférence de tensioactifs exempts d'oxyde d'alkylène et
c) éventuellement 0,01 à 1,0 % en poids d'agent de conservation.

9. Dispersions de cire selon l'une quelconque des revendications 7 ou 8, **caractérisées en ce qu'**elles contiennent

a) 15 à 30 % en poids d'ester de l'éthylène glycol en tant que cire et
b) 10 à 30 % en poids de tensioactifs non ioniques, de préférence choisis parmi des groupes formés d'alkylpolyglucosides et/ou d'alkylènepolyglycosides et
c) éventuellement 0,01 à 0,5 % en poids d'agent de conservation.

10. Dispersions de cire selon l'une quelconque des revendications 7 à 9, **caractérisées en ce qu'**elles contiennent

a) 15 à 30 % en poids d'ester de l'éthylène glycol en tant que cire constituée de a1) 3 à 10 % en poids de monoester de l'éthylène glycol et a2) 90 à 97 % en poids de diester de l'éthylène glycol et
b) 10 à 30 % en poids de tensioactifs non ioniques, choisis parmi des groupes formés d'alkylpolyglucosides et/ou d'alkylènepolyglycosides et
c) éventuellement 0,01 à 0,5 % en poids d'agent de conservation.

11. Dispersions de cire selon l'une quelconque des revendications 7 à 10, **caractérisées en ce qu'**elles contiennent

a) 15 à 30 % en poids d'ester de l'éthylène glycol en tant que cire constituée de 3 à 10 % en poids de monoester et de 90 à 97 % en poids de diester de l'éthylène glycol avec un mélange d'acides gras composé de 40 à 60 % en poids d'acide stéarique et de 40 à 60 % en poids d'acide palmitique et
b) 10 à 30 % en poids de tensioactifs non ioniques, choisis parmi des groupes formés d'alkylpolyglucosides et/ou d'alkylènepolyglycosides et
c) éventuellement 0,01 à 0,5 % en poids d'agent de conservation.

12. Dispersions de cire selon l'une quelconque des revendications 7 à 11, **caractérisées en ce qu'**elles ne contiennent aucun composé pétrochimique.

13. Dispersions de cire selon l'une quelconque des revendications 7 à 12, **caractérisées en ce que** les esters de l'éthylène glycol présentent un point de fusion supérieur à 40 °C, de préférence inférieur à 75 °C et en particulier compris entre 45 et 65 °C.

14. Utilisation des esters selon au moins l'une des revendications 1 à 6 en tant que cire, de préférence sous forme de leurs dispersions selon l'une quelconque des revendications 7 à 13 pour la génération d'une opacité ou d'une brillance nacrée, de préférence pour la génération d'une opacité ou d'une brillance nacrée dans des agents cosmétiques pour le nettoyage de la peau et/ou des cheveux.

15. Utilisation selon la revendication 14, **caractérisée en ce que** les cires sont utilisées sous forme de dispersions de cire selon l'une quelconque des revendications 7 à 13.

16. Agents de nettoyage cosmétiques pour la peau et les cheveux contenant un ester de l'éthylène glycol selon la revendication 1 en tant qu'agent nacrant ou agent opacifiant.

17. Procédé de préparation d'agents de nettoyage cosmétiques selon la revendication 16, **caractérisé en ce que** les esters de l'éthylène glycol selon la revendication 1 sous forme de dispersions selon au moins l'une des revendications 7 à 13 sont ajoutés en mélangeant à température ambiante à un ou plusieurs ingrédients préchargés de l'agent

cosmétique, de préférence à une température comprise entre 15 et 30 °C.

**18.** Procédé de préparation d'agents de nettoyage cosmétiques selon la revendication 16, **caractérisé en ce que** les esters de l'éthylène glycol selon la revendication 1 sont chauffés à des températures supérieures au point de fusion, et sont mélangés avec au moins l'un des ingrédients restants de l'agent cosmétique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19511572
- WO 9838973 A
- WO 2012177886 A
- WO 03066796 A
- EP 1830798 A
- FR 2252840 A


### In der Beschreibung aufgeführte Nicht-Patentliteratur


- **R.LOCHHEAD.** *Cosm.Toil.,* 1993, vol. 108, 95
- Kosmetische Färbemittel. Farbstoffkommission der Deutschen Forschungsgemeinschaft. Verlag Chemie, 1984, 81-106